Europäisches Patentamt

European Patent Office

Office européen des brevets

(19)

(11) Veröffentlichungsnummer: **0 006 607**
**A1**

(12) **EUROPÄISCHE PATENTANMELDUNG**

(21) Anmeldenummer: **79102112.4**

(22) Anmeldetag: **26.06.79**

(51) Int. Cl.³: **A 61 F 13/04**, A 61 L 15/07

(30) Priorität: **27.06.78 DE 2828084**
**16.11.78 DE 2849678**

(43) Veröffentlichungstag der Anmeldung: **09.01.80**
**Patentblatt 80/1**

(84) Benannte Vertragsstaaten: **BE FR IT NL**

(71) Anmelder: **EUROCOM ESTABLISHMENT,**
**Kirchstrasse 37, FL-9490 Vaduz (LI)**

(72) Erfinder: **Kantner, Hans-Joachim, Dipl.-Ing., Am**
**Fichteneck 9, D-6072 Dreieichenhain (DE)**

(74) Vertreter: **Kantner, Hans-Joachim, Dipl.-Ing.,**
**Darmstädter Strasse 8, D-6070 Langen (DE)**

(54) **Bandage zur Unterbindung der Schwenkbeweglichkeit zweier Teile gegeneinander.**

(57) Bandage zur Unterbindung der Schwenkbeweglichkeit zweier Teile gegeneinander, insbesondere zur Ruhigstellung von Gelenken menschlicher oder tierischer Gliedmaßen, wie beispielsweise des Sprunggelenkes eines Beines oder eines Knie- oder Ellenbogengelenkes, relativ zueinander. Die Bandage wird durch einen Abschnitt (2) mindestens einer Lage einer Breitlonguette (3) eines in plastisch verformbarem Zustand auf die einander benachbarten Bereiche der gegeneinander schwenkbeweglichen Teile aufbringbaren Fixiermaterials aus Kunststoff oder von einem biegsamen Trägerkörper abgestützten Fixiermaterial gebildet, das jeweils in diese Bereiche der gegeneinander zu fixierenden Teile umgebenden Stellung zum Aushärten bringbar ist.

EUROCOM ESTABLISHMENT
FL-9490 Vaduz/Liechtenstein

Bandage zur Unterbindung der Schwenkbeweglichkeit
==================================================
zweier Teile gegeneinander
============================

Die Erfindung bezieht sich auf eine Bandage zur Unterbindung der Schwenkbeweglichkeit zweier Teile gegeneinander und betrifft insbesondere eine solche Bandage zur Ruhigstellung von Gelenken menschlicher oder tierischer Gliedmaßen, wie beispielsweise des Sprunggelenks eines Beins oder eines Knie- oder Ellenbogengelenks, relativ zueinander.

Nicht selten kommt es vor, daß sich die Aufgabe nicht nur im medizinischen Bereich stellt, zwei beweglich aneinander angelenkte Teile, wie beispielsweise zwei Streben eines Baugerüsts, so gegeneinander zu fixieren, daß eine vorbestimmte Winkelstellung dieser beiden Teile zueinander gewahrt wird, dabei jedoch die Schwenkbeweglichkeit der Anlenkung derselben aneinander vorübergehend ausgeschlossen wird, beispielsweise weil die betreffende Anlenkung ausgeleiert ist und daher ein nicht mehr zulässiges Spiel aufweist oder in anderer Weise defekt ist. Ein besonders häufiger Anwendungsfall dieser Situation ist die Notwendigkeit der Ruhigstellung menschlicher
oder tierischer Gliedmaßen insbesondere nach Unfällen, wobei
im orthopädischen Bereich in überwiegendem Maße Ruhigstellungen
des Sprunggelenkes des Beins oder aber des Ellenbogens aufzutreten pflegen.

Diese im technischen Bereich bisher unlösbar Aufgabe hat man
im medizinischen Bereich bisher dadurch gelöst, daß man die
gegeneinander zu fixierenden Teile einschließlich ihrer
schwenkbeweglichen Anlenkung aneinander, nämlich beispielsweise Ober- und Unterschenkel und das dazwischenliegende
Kniegelenk oder aber Unterschenkel und Mittelfuß einschließlich des dazwischenliegenden Sprunggelenkes im Knöchelbereich
zirkulär mit bindenförmigem Material umwickelt hat, das als
Trägerkörper für ein Fixiermaterial, wie beispielsweise Gips,
dient.

Abgesehen davon, daß die Anbringung einer solchen Ruhigstellungsbandage mit einem nicht unerheblichen Aufwand an Material
verbunden ist und beträchtliche Verschmutzungen der Umgebung
des Anbringungsortes erfahrungsgemäß unvermeidbar sind, mußte
bisher ein solcher zirkulärer Bindenverband stets von fachärztlichem Personal oder aber entsprechenden orthopädisch
ausgebildeten Hilfskräften für klinische oder ambulante Anbringung solcher Ruhigstellungsverbände . unmittelbar am
Einsatzort angebracht werden. Auch bei spezifischer Fachkunde
des den Ruhigstellungsverband aufbringenden ärztlichen oder
medizinisch-technischen Personals und/oder großer Erfahrung
desselben und/oder hervorragender Aufmerksamkeit desselben
läßt es sich auf Grund der spezifischen Konzeption und Art
eines solchen Ruhigstellungsverbandes erfahrungsgemäß nicht
vermeiden, daß es zu Strikturen und Einschnürungen des vom
Verband abgedeckten Gliedmaßengewebes kommt, welche Anlaß zu zumindest Druckstellen, wenn nicht schlimmeren Schädigungen, wie beispielsweise Nervenquetschungen, sind, die ihrerseits auf Grund insbesondere ihrer Folgeschäden nicht unerhebliche Haftpflichtansprüche auszulösen pflegen. Nachteilig ist weiterhin der notwendige nicht unerhebliche Zeitaufwand für die Anbringung eines solchen bekannten Ruhigstellungsverbandes. Außerdem ist es erfahrungsgemäß kaum möglich, einem solchen Ruhigstellungsverband ein ästhetisch formschönes Äußeres zu geben, welches zudem auch noch frei von Rauhigkeiten ist, an denen sich Schmutzteilchen festsetzen oder aber es zu Verletzungen der unter Umständen höchst feinmaschigen Web- oder Wirkstruktur übergezogener Kleidungsstücke, wie beispielsweise eines Damenstrumpfes, kommen könnte.

Der Erfindung liegt nun die Aufgabe zugrunde, unter Vermeidung der Nachteile bekannter Ruhigstellungsverbände mit höchst einfachen und kostengünstigen Mitteln eine Bandage zur Unterbindung der Schwenkbeweglichkeit zweier Teile gegeneinander, seien dies nun technische Bauteile oder Gelenke menschlicher oder tierischer Gliedmaßen, wie beispielsweise zur Ruhigstellung des Sprungelenkes eines Beins oder eines Knie- oder Ellenbogengelenks, relativ zueinander zu schaffen, welche unter Vermeidung besonderen Schmutzes am Erstellungs- beziehungsweise Einsatzort schnell und bequem nicht nur unmittelbar am Patienten in Arbeitsstellung bringbar ist, sondern auch in höchst wirtschaftlicher Weise bereits vorbereitet in Vorrat gehalten werden kann, um beispielsweise für die Arbeit von Orthopädie-Mechanikern oder orthopädischen Schuhmachern zum Einsatz zu kommen oder aber bei Sportunfällen jederzeit auch so zur Verfügung zu stehen, daß ihr Einsatz nicht auf die Tätigkeit ärztlichen oder medizinisch-technischen und insbesondere orthopädischen Hilfspersonals angewiesen ist, sondern, wie das häufig auf einem Sportfeld, wie etwa einem Fußballplatz, der Fall zu sein pflegt, in erster Hilfe auch von gerade verfügbarem Hilfspersonal, seien dies nun Mannschaftskameraden oder der Trainer oder der Masseur, ohne Gefahr von nicht wieder gutzumachenden Anbringungsfehlern angelegt werden können, wobei insbesondere jegliche Gefahr von Strikturen und dergleichen und damit einhergehender Folgeschäden wirksam ausgeschlossen ist und sich außerdem ohne weiteres auch noch ein besonderes formschönes Äußeres ergibt, das zudem noch praktisch keinerlei Ansatzmöglichkeiten für Schmutz oder Quellen für Beschädigungen von übergezogenen Kleidungsstücken, wie beispielsweise eines Damenstrumpfes besonders feiner Web- oder Wirkstruktur, bietet.

Dies wird durch die Erfindung in überraschend einfacher Weise dadurch erreicht, daß eine Bandage gattungsgemäßer Art durch einen Abschnitt mindestens einer Lage einer Breitlongette eines in plastisch verformbarem Zustand auf die einander benachbarten Bereiche der gegeneinander schwenkbeweglichen Teile aufbringbaren Fixiermaterials aus Kunststoff oder von einem biegsamen Trägerkörper abgestützten Fixiermaterials gebildet ist, das in diese Bereiche der gegeneinander zu fixierenden Teile umgebender Stellung zum Aushärten bringbar ist.

Dabei liegt der Lösungsvariante der Erfindung, die mit Fixiermaterial aus Kunststoff arbeitet, die zusätzliche Aufgabenstellung zugrunde, insbesondere aus Gründen der Vermeidung von Schmutz am Anbringungsort der gattungsgemäßen Bandage und der weitestgehenden Vereinfachung von Lagerhaltung und Anbringung mit der einhergehenden Steigerung der Wirtschaftlichkeit eine Möglichkeit für die Erstellung von Bandagen gattungsgemäßer Art zu schaffen, die es gestattet, die gemäß der grundlegenden, vorstehend beschriebenen Aufgabe zu erzielenden Vorteile wohl zu erreichen und sogar zu übertreffen, dabei aber auf Longettenmaterial zu verzichten, das einen biegsamen Trägerkörper für die Abstützung des in die gegeneinander zu fixierenden Teile umgebender Stellung zum Aushärten bringbaren Fixiermaterials benötigt.

In weiterer zweckmäßiger Fortbildung des mit von einem biegsamen Trägerkörper abgestütztem Fixiermaterial arbeitenden übergeordneten Erfindungsgedankens kann die Bandage nach der Erfindung mindestens vier, vorzugsweise acht Lagen des Breitlongettenmaterials aufweisen.

Gemäß weiterer erfinderischer Fortbildung dieses Erfindungsgedankens kann der Lagenabschnitt durch Abschnitte von gewebtem oder gewirktem Bahnenmaterial mit einer Tränkung oder Beschichtung mit einem unter Feuchtigkeits- und/oder Luft- und/oder Wärmeeinwirkung zu einem praktisch unelastischen Körper aushärtenden Kunststoffmaterial gebildet sein. Dieses hat nämlich neben der besseren Durchlässigkeit für Röntgenstrahlen, welche für die zwischenzeitliche Inspektion beider gegeneinander fixierten Teile ohne Abnahme des erfindungsgemäßen Ruhigstellungsverbandes sehr wünschenswert ist, ein verhältnismäßig geringes Gewicht.

Außerdem weist in den meisten Einsatzfällen eine solche Bandage auch noch den wesentlichen Vorteil auf, daß sie wasserunempfindlicher ist und eine höhere Stabilität gegegenüber Bruchbelastungen erbringen kann als bei Verwendung von Gips als Fixiermaterial. Andererseits kann es gemäß einer Alternativlösung hierzu insbesondere aus Kostengründen oder auf Grund gerade am Einsatzort verfügbaren Grundstoffmaterials durchaus auch zweckmäßig sein, wenn der Lagenabschnitt durch Abschnitte von an sich bekanntem gewebtem oder gewirktem Bahnenmaterial mit Gipsbeschichtung gebildet ist.

Gemäß einer erfinderischen Fortbildung des mit Fixiermaterial aus Kunststoff arbeitenden anderen untergeordneten Erfindungsgedankens kann der Lagenabschnitt aus unter Feuchtigkeits- und/oder Luft- und/oder Wärmeeinwirkung zu einem praktisch unelastischen Körper
aushärtendem Kunststoffmaterial oder alternativ hierzu
aus unter Feuchtigkeits- und/oder Luft- und/oder Wärmeeinwirkung in plastisch verformbaren Zustand verbringbarem Kunststoffmaterial gebildet sein. Dieses hat nämlich ebenso wie bei der entsprechenden Fortbildung des
erstbeschriebenen übergeordneten Erfindungsgedankens
neben der besseren Durchlässigkeit für Röntgenstrahlen,
welche für die zwischenzeitliche Inspektion beider gegeneinander fixierten Teile ohne Abnahme des erfindungsgemäßen Ruhigstellungsverbandes sehr wünschenswert ist,
ein verhältnismäßig geringes Gewicht. Außerdem weist
auch hier in den meisten Einsatzfällen eine solche
Bandage auch noch den wesentlichen Vorteil auf, daß
sie wasserunempfindlicher ist und eine höhere Stabilität gegenüber Bruchbelastungen erbringen kann als
bei Verwendung von Gips als Fixiermaterial.

Als besonders zu bevorzugen hat es sich ungeachtet
der speziellen Ausführungsform der Erfindung gemäß
deren einem, mit von einem biegsamen Trägerkörper
abgestütztem Fixiermaterial arbeitendem übergeordnetem Erfindungsgedanken oder deren anderem, mit
Fixiermaterial aus Kunststoff arbeitendem übergeordnetem Erfindungsgedanken herausgestellt, wenn
Breitlongettenmaterial einer Breite von etwa 40 bis
60 cm verwandt wird, da je nach Bedarf in Abschnitten
einer Länge von 70 bis 80 cm vom Lagenvorrat abgeschnitten wird.

Grundsätzlich kann der erfindungsgemäße Lagenabschnitt in unveränderter Form um die beiden gegeneinander zu fixierenden Teile, beispielsweise
den Unterschenkel und Fuß eines menschlichen
Beins samt dem dazwischenliegenden Sprunggelenk, herummodelliert werden. Dabei werden
sich allerdings meist nicht ohne weiteres
Verdickungen beispielsweise im Knöchelbereich,
allgemein gesprochen im Bereich der Schwenkbeweglichkeit der gegeneinander zu fixierenden
Teile, vermeiden lassen. Bei der Fixierung
technischer Bauteile in vorbestimmter Winkelstellung zueinander pflegt dies keinen Nachteil
darzustellen und daher unbeachtlich zu sein.
Bei der Ruhigstellung menschlicher Gliedmaßen
hingegen können je nach Art und weiterem Bestimmungszweck der Ruhigstellungsbandage solche
Verdickungen im Knöchelbereich höchst störend sein.
Aus diesem Grunde sieht eine weitere erfinderische
Fortbildung vor, daß der Lagenabschnitt bzw.
die Lagenabschnitte im Bereich der Schwenkbeweglichkeit der gegeneinander zu fixierenden
Teile jeweils einen von ihrer Randkontur
etwa zu ihrer Mittellängslinie verlaufenden
Schlitz aufweist. Auf diese Weise können nämlich statt des sonst notwendigen Übereinanderlegens des seitlich überschüssigen Bahnenmaterials in Zickzackanordnung diese Bahnenmaterialbereiche einander lediglich einschichtig
überdeckend zur Aussteifung des Bereiches der Schwenkbeweglichkeit der gegeneinander zu fixierenden Teile, beispielsweise also des Knöchelbereichs, herangezogen werden. Andererseits kann es aber auch erwünscht sein, in diesem Bereich, beispielsweise dem Knöchelbereich, keine Verdickungen überhaupt zu erhalten, wie das beispielsweise dann der Fall sein kann, wenn die erfindungsgemäße Ruhigstellungsbandage im betreffenden spezifischen Einsatzfall als Einziehstütze zur Verwendung kommen soll, über die dann noch ein Strumpf oder Socken und anschließend ein geschlossener Schuh oder Stiefel gezogen werden soll. In einem solchen Fall sieht eine alternative erfinderische Fortbildung vor, daß der Lagenabschnitt bzw. die Lagenabschnitte im Bereich der Schwenkbeweglichkeit der gegeneinander zu fixierenden Teile jeweils eine von ihrer Randkontur etwa zu ihrer Mittellängslinie verlaufende Materialausnehmung aufweist bzw. aufweisen. Dabei kann in weiterer zweckmäßiger Fortbildung die Materialausnehmung des Lagenabschnittes bzw. der Lagenabschnitte sich etwa keilförmig zu deren Mittellängslinie hin verjüngende Form aufweisen. Es ist erkennbar, daß bei einer Ausführung der erfindungsgemäßen Bandage gemäß diesem untergeordneten Erfindungsgedanken sich ein praktisch einlagiges Winkelröhrengebilde als Ruhigstellungsstütze für die beiden gegeneinander zu fixierenden Teile praktisch ohne Verdickungen im Bereich von deren Schwenkbeweglichkeit ergibt.

Weiterhin hat es sich aus Gründen der Vermeidung
unerwünschter Verdickungen als höchst zweckmäßig
erwiesen, wenn gemäß einem anderen die Erfindung
in nicht naheliegender Weise fortbildenden untergeordneten Erfindungsgedanken der Lagenabschnitt
bzw. die Lagenabschnitte beidseitig des Bereichs
der Schwenkbeweglichkeit der gegeneinander zu
fixierenden Teile jeweils eine Einengung ihrer
seitlichen Randkontur aufweist bzw. aufweisen.
Dabei hat es sich als besonders zweckmäßig
herausgestellt, wenn eine erste Einengung mit
einer geringsten Materialbreite in einem Abstand von der oberen Begrenzungskontur von
etwa 30 bis 40 cm und/oder eine zweite Einengung mit einer geringsten Materialbreite in
einem Abstand von der oberen Begrenzungskontur
von etwa 50 bis 60 cm vorgesehen ist.

In der Regel werden Ruhigstellungsbandagen
beschriebener Art so eingesetzt, daß sie an den
in der gewünschten Winkelstellung zueinander
zu fixierenden Teilen, wie beispielsweise
menschlichen oder tierischen Gliedmaßen,
solange verbleiben, bis der Ruhigstellungszweck erfüllt ist, um danach abgenommen und
beseitigt zu werden, wobei das Abnehmen der
Arbeitsersparnis wegen meist mit einer Zerstörung der Bandage einhergeht. So hat sich
hier die Lösung gemäß einem die mit Fixiermaterial
aus Kunststoff arbeitende Ausführungsform einer erfindungsgemäßen Bandage zweckmäßig fortbildenden
untergeordneten Erfindungsgedanken besonders bewährt, gemäß welchem die bei die gegeneinander
schwenkbeweglichen Teile umschließender Arbeitsstellung einander benachbarten Längsrandbereiche eines jeden Lagenabschnitts miteinander
durch Verschweißen, Verkleben, Verseifung oder
dergleichen verbunden sind. Dabei kann es durchaus
empfehlenswert sein, und zwar aus Gründen beispielsweise einer etwa erwünschten Nachkorrektur der Paßform der Bandage, wenn die Verbindung beider Lagen-
Längsrandbereiche miteinander durch Wiederaufbringung von Feuchtigkeits- und/oder Luft- und/oder
Wärmeeinwirkung beispielsweise mittels Auflegens
feucht-heißer Kompressen wieder in plastisch verformbaren Zustand zurückverbringbar ist.

Andererseits gibt es jedoch Einsatzfälle, wo es
erwünscht ist, eine solche Bandage ohne Veränderung
ihrer Paßform zwischenzeitlich abnehmen zu können,
um beispielsweise den in Ruhigstellung zu haltenden
Gliedmaßen etwa zur Schlafenszeit die Möglichkeit
zu geben, belastungsfrei unter natürlichen Atmungs-
und Hautreizungsbedingungen auszuruhen. Erwünscht
ist dies bei Ellenbogenverbänden und insbesondere
bei sogenannten Gehverbänden, bei denen Unterschenkel und Mittelfuß einschließlich des zwischenliegenden Sprunggelenkes in vorgegebener Stellung so
gegeneinander fixiert zu halten sind, daß das
Sprunggelenk keinerlei Bewegungsmöglichkeit hat.
Häufig kann dieser Fall bei Sportunfällen auftreten,
bei denen es gilt, Zerrungen und/oder Verstauchungen
im Bereich des Sprunggelenkes durch Ruhigstellung
mit Unterbindung der Bewegungsmöglichkeit der betreffenden miteinander zusammenwirkenden Gliedmaßen zu
kurieren.

Bei Dorsalschienen für die Ruhigstellung des Arms im Ellenbogenbereich hat man bereits eine Abnehmbarkeit einer solchen
Schiene vorgesehen. Diese Abnehmbarkeit wird dadurch erreicht, daß die am Arm angelegte Schiene durch Umwickeln
von Arm und Schiene mittels einer Mullbinde oder dergleichen
am Arm gehalten wird. Diese Art der Anbringung ist höchst
umständlich und arbeitsaufwendig, zumal es nicht damit getan
ist, durch Abwickeln des Bindenmaterials die Schiene vom
Arm zu lösen, sondern bekanntlich das Bindenmaterial dann
entweder weggeworfen werden muß, was einen nicht unbeträchtlichen wirtschaftlichen Verlust bedeutet und besondere Abfallbeseitigungsprobleme mit sich zu bringen pflegt, oder
aber durch Aufwickeln in benutzungsbereiten Zustand für die
nächste Festlegung der Schiene am Arm verbracht werden muß.
Diese Art einer lösbaren Festlegung einer Schiene am Arm
ist im übrigen für eine Übertragung auf einen Gehverband
an einem Bein ungeeignet. Einem die Erfindung zweckmäßig
fortbildenden weiteren untergeordneten Erfindungsgedanken
liegt nunmehr die zusätzliche Aufgabe zugrunde, die erfindungsgemäße Bandage so auszugestalten, daß mit ihr erstmalig auch ein Gehverband oder dergleichen realisiert werden
kann, der schnell und bequem und dabei auch noch frei von
Schmutz und Abfall und zusätzlichem Arbeitsaufwand an den
in Ruhigstellung zueinander zu fixierenden Teilen, wie beispielsweise dem Unterschenkel und Fuß eines menschlichen
Beins, angelegt und ebenso schnell und bequem wieder abgenommen werden kann, wenn dies erwünscht ist, im übrigen aber
seine erfindungsgemäßen günstigen Stabilitätseigenschaften
mit deren vorteilhafter Auswirkung auf das Ruhigstellungsverhalten der gegeneinander zu fixierenden Teile unvermindert beibehält.

Die Lösung dieses Erfindungsgedankens kennzeichnet sich dadurch, daß längs beider Längsrandkonturen aller Lagenabschnitte jeweils der Randstreifen einer Hälfte einer teilbaren Verschlußleiste, wie beispielsweise eines teilbaren Reißverschlusses, Klettverschlusses oder Haken- und/oder Ösenverschlusses, festgelegt ist.

In weiterer zweckmäßiger Fortbildung können die Randstreifen der beiden Hälften der teilbaren Verschlußleiste jeweils unter Zwischenschaltung einer Longette fixiermaterialfreien Materials, vorzugsweise Textilmaterials, festgelegt sein. Durch eine solche Longette wird die Möglichkeit eröffnet, die schlitzförmige Entnahmeöffnung des erfindungsgemäßen Ruhigstellungsverbandes ausreichend breit bemessen zu können, ohne daß der Verband an Stützfestigkeit für die ruhigzustellenden Teile beziehungsweise Gliedmaßen leidet, da die gesamte Entnahmeöffnungsbreite durch die in Schließstellung befindliche Verschlußleiste und deren randseitige Longetten satt und stramm überdeckt wird.

Die Festlegung der Verschlußelemente kann in weiterer zweckmäßiger Fortbildung dadurch geschehen, daß die Randstreifen der beiden Hälften der teilbaren Verschlußleiste oder die Longetten fixiermaterialfreien Materials jeweils durch den Aushärtungshärtungsvorgang des Fixiermaterials an diesem festgelegt werden, es sind aber durchaus auch Ausführungsformen möglich, bei denen die Randstreifen der beiden Hälften der teilbaren Verschlußleiste oder die Longetten fixiermaterialfreien Materials jeweils mechanisch am Trägerkörper des Fixiermaterials mindestens einer Lage, vorzugsweise aber aller Lagen, festgelegt sind. Zweckmäßigerweise kann diese Festlegung im Falle der Ausführung des Materials der aneinander festzulegenden Teile in Kunststoff durch Verschweißen oder Verkleben erfolgen, meist wird sich jedoch Vernähen als geeigneter erweisen, und zwar dies insbesondere immer dann, wenn eines der aneinander festzulegenden Teile aus nicht verschweißbarem Textilmaterial besteht. Auch für nicht verschweißbares Material kann sich Verkleben als geeignete
Verbindungsart empfehlen.

Es hat sich im übrigen herausgestellt, daß bevorzugt der
durch den Randstreifen der Verschlußleiste und/oder eine
etwa zwischengeschaltete Longette gebildete fixiermaterialfreie Streifenbereich eine einseitige Breite von mindestens
2 cm, vorzugsweise 3 cm, aufweisen sollte. Hierdurch wird
einerseits praktisch jegliche Beeinträchtigung der Festigkeits- und Steifigkeitseigenschaften des Stützverbandes
vermieden, im übrigen aber eine für die Entnahme praktisch
jeder üblichen Größe beziehungsweise Dicke zu behandelnder
Gliedmaßen ausreichend breite schlitzförmige Entnahmeöffnung
geschaffen.

Es ist ersichtlich, daß sich die Erfindung durch besonders
geringe Zahl von Einzelteilen und geringen Herstellungsaufwand und damit einhergehend einen extrem günstigen Gestehungspreis trotz besonderer Universalität der Einsatz- und Anwendungsmöglichkeiten auszeichnet. Es braucht nämlich lediglich eine einziges Breitlongettenmaterial geeigneter Lagenbreite auf die entsprechend der Größe beziehungsweise Länge
der zu behandelnden Gliedmaßen erforderliche Länge geschnitten und gegebenenfalls in geeigneter, vorstehend beschriebener
Weise konturiert zu werden und gewünschtenfalls mit einer
teilbaren Verschlußleiste geeigneter Länge und Ausführung
zur Verwendung zu kommen, indem alle weiteren funktionswichtigen Eigenschaften der erfindungsgemäßen Stützbandage durch
geeignetes Anmodellieren derselben an den betreffenden zu
fixierenden Teilen, beispielsweise den gegeneinander ruhigzustellenden menschlichen oder tierischen Gliedmaßen, hervorgebracht werden.

Im folgenden wird die Erfindung anhand eines Ausführungsbeispiels, das in den Zeichnungen schematisch dargestellt ist,
rein beispielsweise näher beschrieben. Dabei zeigen:

Figur 1 eine Seitenansicht eines mit einer Ruhigstellungsbandage nach der Erfindung versehenen Beins von dessen
Innenseite her, und

Figur 2 eine Draufsicht auf einen für die Erstellung der Ruhigstellungsbandage nach der Erfindung besonders geeigneten Breitlongetten-Abschnitt.

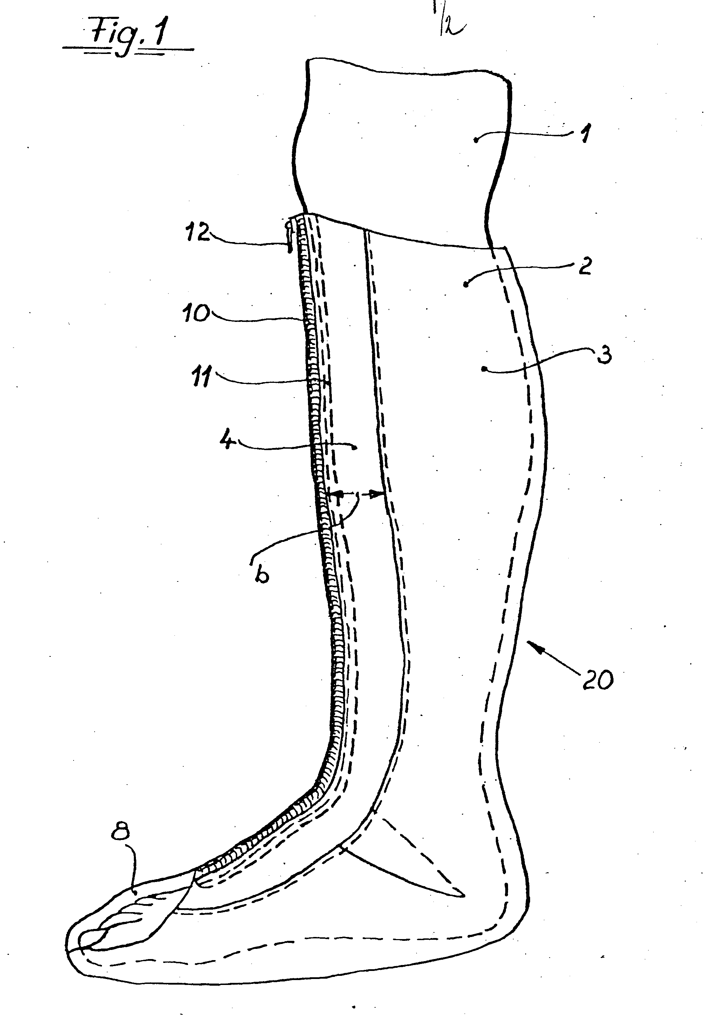

Das mit dem als Ganzes mit 20 bezeichneten Unterschenkelgehverband nach der Erfindung versehene Bein ist in Figur 1 mit
1 bezeichnet. Die Zehenöffnung dieses Gehverbandes ist mit 8
bezeichnet. An diesem Gehverband 20 kann bei Bedarf auf dessen
die Fußsohle abdeckender Unterseite in geeigneter Weise eine
Laufsohle oder Rollstütze festgelegt werden, um diese Unterseite des Gehverbandes 20 vor übermäßiger Abnutzung zu schützen
und andererseits, sollte dies aus orthopädischen Gründen zweckmäßig sein, für ein gutes Lauf- bzw. Gehverhalten des Patienten zu sorgen.

Die dargestellte Bandage 20 ist als Abschnitt 2 einer Mehrfachlage von vorzugsweise achtlagig liegendem Breitlongettenmaterial 3 gebildet. Dabei kann das Breitlongettenmaterial
zum Beispiel gewebtes oder gewirktes Bahnenmaterial als Trägerkörper für eine Tränkung oder Beschichtung mit einem Kunststoffmaterial sein, das unter Feuchtigkeits- und/oder Luft-
und/oder Wärmeeinwirkung zu einem praktisch unelastischen
Körper aushärtet, andererseits kann es aber durchaus zu bevorzugen sein, wenn dieses Breitlongettenmaterial 3 durch
einen Trägerkörper aus gewebtem oder gewirktem Material
nach Art einer üblichen Gipsbinde gebildet ist, die eine
Gipsbeschichtung aufweist.

Andererseits kann die dargestellte Bandage 20 aber auch als
Abschnitt 2 einer Einfachlage von Breitlongettenmaterial 3
aus Kunststoff gebildet sein. Bevorzugt wird dabei ein solches
Kunststoffmaterial, das unter Feuchtigkeits- und/oder Luft-
und/oder Wärmeeinwirkung in plastisch verformbaren Zustand
verbringbar ist, bevor es nach dem Anmodellieren an den gegeneinander zu fixieren Teilen der Gliedmaßen in der gewünschten
Arbeitsstellung zum Aushärten gebracht wird.

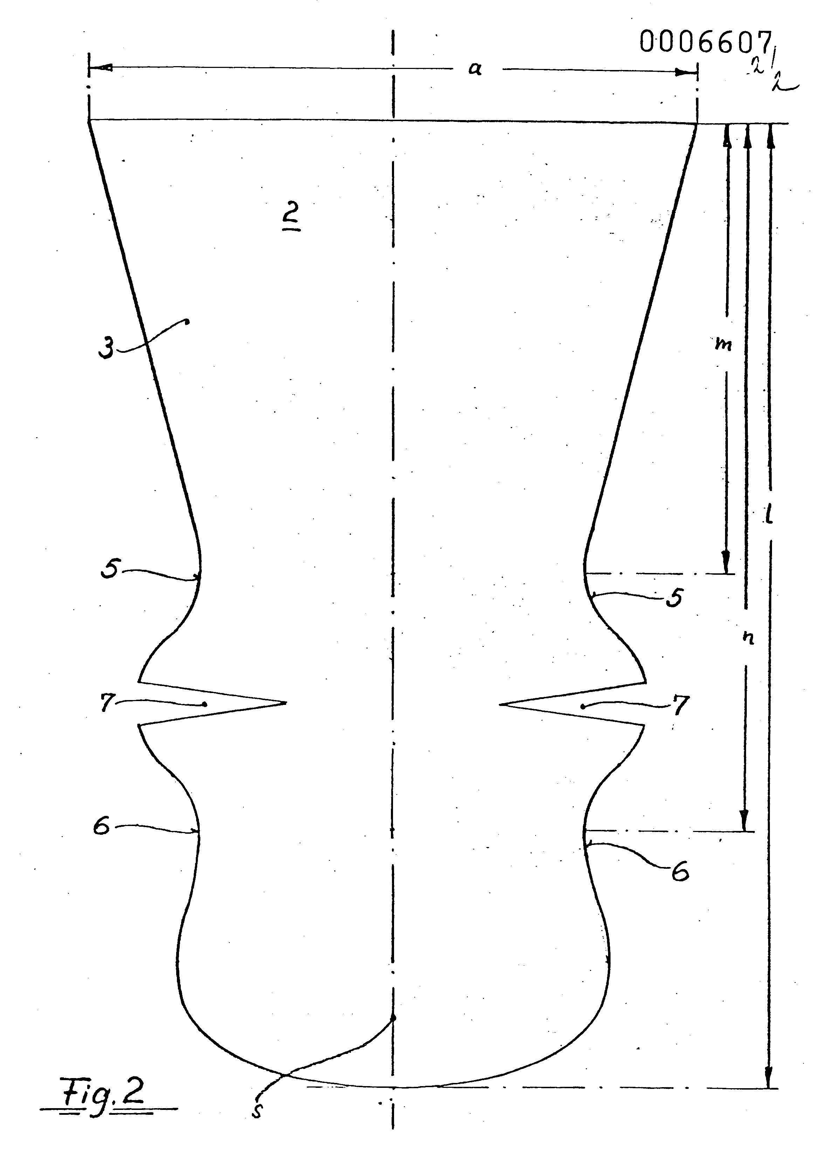

Der in Figur 2 dargestellte Bandagenrohling in Form eines Abschnittes 2 von ein- oder mehrlagigem Breitlongettenmaterial 3
weist eine Breite a der Breitlongette 3 von 40 bis 60 cm
und eine Gesamtlänge l von etwa 70 bis 80 cm auf. Obgleich,
wie eingangs bereits dargelegt, ein solcher Bandagenrohling
für den erfindungsgemäßen Zweck auch in bezüglich seiner
Randkanten nicht konturiertem Zustand, also als reiner
Abschnitt des Lagenmaterials, zum Einsatz kommen kann,
sollte er zweckmäßig doch in dem Bereich, der beim dargestellten Ausführungsbeispiel etwa den Knöchel und damit
auch das Sprunggelenk des ruhigzustellenden Beins 1 umfaßt, einen von seiner Randkontur zu seiner Mittellängslinie s verlaufenden Schlitz aufweisen. Dieser kann zweckmäßig jeweils als sich zur Mittellängslinie s hin keilförmig verjüngende Ausnehmung 7 ausgebildet sein, wie in Figur 2 dargestellt. Auf diese Weise lassen sich Bandagen erstellen, die im Knöchelbereich beziehungsweise allgemein
im Bereich der Schwenkbeweglichkeit der gegeneinander zu
fixierenden Teile extrem verdickungsfrei sind. Beim in
Figur 1 dargestellten Ausführungsbeispiel ist eine leichte
Überlappung des unterschenkelseitigen Oberteils der im übrigen
einstückigen Bandage nach der Erfindung über den fußseitigen
Unterteil dargestellt.

Weiterhin weist das in Figur 2 dargestellte Ausführungsbeispiel an jeder seiner beiden Längsrandkonturen jeweils eine
obere und als erste bezeichnete Einengung 5 mit einer geringsten Materialbreite in einem Abstand m von der oberen
Begrenzungskontur von etwa 30 bis 40 cm und eine untere,
als zweite bezeichnete Einengung 6 mit einer geringsten
Materialbreite in einem Abstand n von der oberen Begrenzungskontur von etwa 50 bis 60 cm auf.

Der den Bereich der Schwenkbeweglichkeit der gegeneinander
zu fixierenden Teile, beim in Figur 1 dargestellten Einsatzfall den Knöchelbereich des Beins umgreifende Teil
des Breitlongettenrohlings 2 liegt zwischen diesen beiden
Einengungsbereichen 5 und 6.

Der auf diese Weise gebildete Bandagenrohling kann in geeigneter Stückzahl, Größenausführung und/oder Randkonturierung
seiner beiden Längsränder, die im übrigen keineswegs symmetrisch zur Mittellängslinie s ausgebildet zu sein braucht,
sondern durchaus auch ungleich sein kann, vorgefertigt und
auf Lager gehalten werden, um im Bedarfsfall sofort zur
Verfügung zu stehen. Diese Bandagenrohlinge sind in ähnlicher Weise wie Tücher, Taschentücher oder Socken in
flachgelegter Stellung auf geringstem Raum in ausreichender
Zahl stapelbar. Im Bedarfsfall wird einer der flachgelegten
Bandagenrohlinge in Benutzung genommen, indem er in plastisch
verformbaren Zustand verbracht wird, wie beispielsweise bei
einer Ausführung in Gipsbindenmaterial durch entsprechendes
Anfeuchten, und dann in diesem Zustand um die in Ruhestellung zu fixierenden Gliedmaßen, im Falle des dargestellten
Ausführungsbeispiels sowohl um den Unterschenkel, als auch
den Mittelfuß und den zwischen beiden liegenden Knöchelbereich, lappenförmig herumgelegt und längs der freien Randkanten fixiert wird. Sowohl Gips als auch Kunststoffmaterial,
das unter Feuchtigkeits- und/oder Luft- und/oder Wärmeeinwirkung zu einem praktisch unelastischen Körper aushärtet,
wie beispielsweise Polyurethan, vermitteln dem Bandagenrohling ein feucht-weiches und schmiegsames Verhalten, auf
Grund dessen er lappenartig ausgezeichnet um die gegeneinander ruhigzustellenden Gliedmaßen zu modellieren ist.
Ferner durchtränken beide Materialien das gewirkte Material
der den Bandagenrohling bildenden Lagenabschnitte, so daß
sich, sollte diese Art eines Gehverbandes beabsichtigt sein,
die freien Längsrandbereiche des Bandagenrohlings etwa
längs des Schienenbeins und des Fußristes übereinander
legen und ohne weitere Hilfsmittel miteinander verkleben
lassen.

Wird einlagiges Longettenmaterial aus Kunststoff
als Fixiermaterial verwandt, dann bedarf es keines
Trägermaterials, dennoch aber läßt sich das gleiche
feucht-weiche und schmiegsame Verhalten dieses Materials
in gleicher Weise vorteilhaft nutzen, und zwar dies auch
mit dem Vorteil der Möglichkeit der Verbindung der freien
Längsrandbereiche des Bandagenrohlings ohne weitere Hilfsmittel lediglich durch Verkleben oder dgl. Im übrigen kann
statt Polyurethan auch eine Vielzahl anderer Kunststoffmaterialien zur Verwendung kommen, und zwar für die mit
Longettenmaterial aus Kunststoff arbeitende Variante
der Erfindung bevorzugt durch chemische und/oder Vernetzungs-Reaktion von Polyvinylalkohol mit Formaldehyd
erhaltener Polyvinylalkoholformalschaum. Bei diesem sind
allerdings für die Auslegung des Bandagenrohlings in
besonderem Maße die Schwundeigenschaften dieses Materials
unter insbesondere Feuchtigkeits- und/oder Temperatureinwirkung zu beachten. Weiterhin kann es zur Vereinfachung
des Anlegens der Bandage und damit einhergehend zur Steigerung der Wirtschaftlichkeit des gesamten Einsatzes
derselben durchaus zweckmäßig sein, wenn der Bandagenrohling bereits auf ein bestimmtes Maß vorgeformt, also
mit einer gewissen vorbestimmten Wölbung zur Verfügung
steht, um dann nur noch seine spezielle Anpassungsverformung an den jeweiligen spezifischen Einsatzfall entsprechend den jeweiligen Abmessungen der gegeneinander
ruhigzustellenden Gliedmaßen des Patienten und dem jeweils gewünschten Ruhigstellungswinkel zu erhalten.

Im Interesse einer Steigerung der Steifigkeit der fertiggestellten Bandage ohne zusätzliche Wandstärkenvergrößerung
kann es im übrigen zweckmäßig sein, den Bandagenrohling
entsprechend der vorbestimmten Formgebung im Gießverfahren
zu erstellen, statt ihm durch Biegeverformung eines ebenen
Rohlingsabschnittes auf diese Vorverformungsgestalt zu bringen.

Das Ergebnis ist dann in jedem Fall ein fester winkelröhrenförmiger Verbandverbund. Dieser hat solange am Bein zu bleiben,
wie dieses in Ruhigstellung gehalten werden soll. Danach
wird er durch Zerstörung, beispielsweise durch Auftrennen
in mehrere Teile, abgenommen.

Soll ein Gehverband geschaffen werden, der jederzeit beliebig vom Bein abnehmbar ist, so kann - wie beim in Figur
1 dargestellten Ausführungsbeispiel - eine teilbare Verschlußleiste vorgesehen sein. Diese kann beispielsweise als
teilbarer Reißverschluß, teilbarer Klettverschluß oder aber
auch als teilbarer Haken- und/oder Ösenverschluß ausgeführt sein. Beim dargestellten Ausführungsbeispiel ist ein
teilbarer Reißverschluß 10 mit einem Betätigungsgriff 12
verwandt. Die Randstreifen 11 der beiden Reißverschlußhälften
können an sich unmittelbar längs der nach dem Umlagen des
feucht-weichen lappenartigen Bandagenrohlings um die betreffenden Gliedmaßenbereiche vorderseitigen Randkantenkontur
festgelegt sein, wobei zweckmäßig diese Randstreifen 11 in
den sich miteinander verklebenden Lagenverbund eingefügt
und dort durch das Aushärten des Fixiermaterials am Verbandverbund selbsttätig festgelegt werden.

Beim dargestellten Ausführungsbeispiel ist jedoch der Reißverschluß 10 mit seinen Randstreifen 11 über jeweils eine
zwischengeschaltete Longette 4 aus nicht mit Fixiermaterial
durchtränktem Textilmaterial am durch den Bandagenrohling 2
gebildeten Schalenverbund festgelegt. Dabei entspricht jede
der beiden fixiermaterialfreien Longetten 4 in ihrer Länge
etwa der Länge des Reißverschlusses, die im übrigen annähernd
der Länge der Längsrandkontur des Bandagenrohlings 2 entspricht. Demgemäß könnte durchaus die Longette 4 jeweils
randseitig längs der Randkontur des Bandagenrohlings 2 bereits vor dessen Anbringung am ruhigzustellenden Bein oder
dergleichen mechanisch festgelegt sein, auf Grund des bevorzugt vorzusehenden Schlitzes beziehungsweise der entsprechenden Materialausnehmung 7 ist es aber zu bevorzugen, wenn diese Longette 4 jeweils am zugeordneten Randstreifen 11 des Reißverschlusses 10 oder dergleichen mechanisch, beispielsweise durch Verkleben, Anschweißen, Annähen oder Anvulkanisieren, festgelegt ist, so daß dann lediglich ihr bandagenrohlingsseitiger Randbereich nach der Umkleidung des ruhigzustellenden Beins mit dem Bandagenrohling 2 durch Einfügen in dessen Lagenverbund und Verklebung mit diesem im Zuge des Aushärtungsvorganges seines Fixiermaterials fest verbunden wird, wodurch gewährleistet ist, daß stets eine etwaige Überlappung einzelner Bereiche in der Nachbarschaft der knöchelseitigen Schlitze beziehungsweise der entsprechenden Ausnehmungen 7 längenmäßig berücksichtigt sind.

Die Breite der Longette 4 wird dabei zweckmäßig so gewählt, daß der durch den Randstreifen 11 des teilbaren Reißverschlusses 10 und der Longette 4 gebildete fixiermaterialfreie Streifenbereich eine einseitige Breite b von mindestens 2 cm, vorzugsweise 3 cm, aufweist.

Auf jeden Fall wird bei dieser Ausführungsform mit beidseitig des Reißverschlusses 10 zwischengeschalteten Longetten fixiermaterialfreien Materials eine von oben nach unten verlaufende Längsöffnung im Bandagenverbund 20 geschaffen, die nicht durch steif fixiertes Material, sondern durch fixiermaterialfreies Material in Form der jeweiligen Longette 4 im Verein mit der Breite des Randstreifens 11 der Verschlußleiste, beispielsweise des dargestellten Reißverschlusses 10, überdeckt wird, und zwar eine etwa schlitzförmige Entnahmeöffnung für das Trennen von Bein und Bandagenverbund 20 ausreichender Breite darbietet, dabei jedoch ohne Beeinträchtigung der Festigkeit und Steifigkeit und damit Stützfähigkeit dieses Bandagenverbundes 20 die schienenbeinseitigen Vorderbereiche von Unterschenkel und Fuß satt und stramm übergreift.

0006607

Obgleich die Erfindung am speziellen Beispiel eines Unterschenkelgehverbandes näher erläutert worden ist, ist sie ersichtlich nicht auf dieses beschränkt. Sie kann in vielfältiger Weise auch für die winkelmäßige Fixierung technischer Bauteile Anwendung finden. Hervorzuheben ist, daß auch andere Ruhigstellungswinkel als der beim dargestellten Ausführungsbeispiel gegebene Winkel von etwa $90^{\circ}$ zwischen Mittelfuß und Unterschenkel sich mittels der Erfindung in gleicher vorteilhafter Weise verwirklichen lassen. Insbesondere kann die Erfindung durch andere Kombination ihrer Merkmale oder deren Austausch gegen gleichwertige Mittel an die jeweiligen Eigenschaften der zur Verfügung stehenden Materialien und die Forderungen des jeweiligen Einsatzfalls angepasst werden, ohne dadurch den Rahmen der Erfindung zu verlassen.

0006607

<div style="text-align:center">

**A n s p r ü c h e**
==================

</div>

1.) Bandage zur Unterbindung der Schwenkbeweglichkeit zweier
Teile gegeneinander, insbesondere zur Ruhigstellung von
Gelenken menschlicher oder tierischer Gliedmaßen, wie beispielsweise des Sprunggelenkes eines Beins oder eines Knie-
oder Ellenbogengelenks, relativ zueinander, dadurch gekennzeichnet, daß sie durch einen Abschnitt (2) mindestens einer
Lage einer Breitlongette (3) eines in plastisch verformbarem Zustand auf die einander benachbarten Bereiche der
gegeneinander schwenkbeweglichen Teile aufbringbaren Fixiermaterials aus Kunststoff oder von einem biegsamen Trägerkörper abgestützen Fixiermaterials gebildet ist, das jeweils
in diese Bereiche der gegeneinander zu fixierenden Teile umgebender Stellung zum Aushärten bringbar ist.

2.) Bandage nach Anspruch 1 mit von einem biegsamen Trägerkörper
abgestützem Fixiermaterial, dadurch gekennzeichnet, daß sie
mindestens vier, vorzugsweise acht Lagen des Breitlongettenmaterials aufweist.

3.) Bandage nach Anspruch 1 oder 2 mit von einem biegsamen Trägerkörper abgestützem Fixiermaterial, dadurch gekennzeichnet, daß
der Lagenabschnitt (2) durch Abschnitte von gewebtem oder
gewirktem Bahnenmaterial mit einer Tränkung oder Beschichtung

<div style="text-align:right">- 2 -</div>

mit einem unter Feuchtigkeits- und/oder Luft- und/oder Wärmeeinwirkung zu einem praktisch unelastischen Körper aushärtenden Kunststoffmaterial gebildet ist.

4.) Bandage nach Anspruch 1 oder 2 mit von einem biegsamen Trägerkörper abgestütztem Fixiermaterial, dadurch gekennzeichnet, daß der Lagenabschnitt (2) durch Abschnitte von an sich bekanntem gewebtem oder gewirktem Bahnenmaterial mit Gipsbeschichtung gebildet ist.

5.) Bandage nach Anspruch 1 mit Fixiermaterial aus Kunststoff, dadurch gekennzeichnet, daß der Lagenabschnitt (2) aus unter Feuchtigkeits- und/oder Luft- und/oder Wärmeeinwirkung zu einem praktisch unelastischen Körper aushärtendem Kunststoffmaterial gebildet ist.

6.) Bandage nach Anspruch 1 oder 5 mit Fixiermaterial aus Kunststoff, dadurch gekennzeichnet, daß der Lagenabschnitt (2) aus unter Feuchtigkeits- und/oder Luft- und/oder Wärmeeinwirkung in plastisch verformbaren Zustand verbringbarem Kunststoffmaterial gebildet ist.

7.) Bandage nach einem der vorhergehenden Ansprüche, gekennzeichnet durch eine Breite (a) der Breitlongette (3) von etwa 40 bis 60 cm.

8.) Bandage nach einem der vorhergehenden Ansprüche, gekennzeichnet durch eine Länge (1) des bzw. der Lagenabschnitte(s) (2) von etwa 70 bis 80 cm.

9.) Bandage nach einem der vorhergehenden Ansprüche, dadurch gekennzeichnet, daß der bzw. die Lagenabschnitte(e) (2) im Bereich der Schwenkbeweglichkeit der gegeneinander zu fixierenden Teile jeweils einen von ihrer Randkontur etwa zu ihrer Mittellängslinie(s) verlaufenden Schlitz aufweist bzw. aufweisen.

10.) Bandage nach einem der vorhergehenden Ansprüche, dadurch
gekennzeichnet, daß der bzw. die Lagenabschnitte(e) (2)
im Bereich der Schwenkbeweglichkeit der gegeneinander zu
fixierenden Teile jeweils eine von ihrer Randkontur etwa
zu ihrer Mittellängslinie (s) verlaufende Materialausnehmung
(7) aufweist bzw. aufweisen.

11.) Bandage nach Anspruch 10, dadurch gekennzeichnet, daß
die Materialausnehmung (7) des Lagenabschnitts bzw. der
Lagenabschnitte (2) sich etwa keilförmig zu deren Mittellängslinie (s) hin verjüngende Form aufweist.

12.) Bandage nach einem der vorhergehenden Ansprüche, dadurch
gekennzeichnet, daß der bzw. die Lagenabschnitte(e) (2)
beidseitig des Bereichs der Schwenkbeweglichkeit der gegeneinander zu fixierenden Teile jeweils eine Einengung (5 bzw.
6) ihrer seitlichen Randkontur aufweist bzw. aufweisen.

13.) Bandage nach Anspruch 12, dadurch gekennzeichnet, daß eine
erste Einengung (5) mit einer geringsten Materialbreite in
einem Abstand (m) von der oberen Begrenzungskontur von etwa
30 bis 40 cm vorgesehen ist.

14.) Bandage nach Anspruch 12 oder 13, dadurch gekennzeichnet, daß
eine zweite Einengung mit einer geringsten Materialbreite in
einem Abstand (n) von der oberen Begrenzungskontur von etwa
50 bis 60 cm vorgesehen ist.

15.) Bandage nach einem der vorhergehenden Ansprüche mit Fixiermaterial aus Kunststoff, dadurch gekennzeichnet, daß die
bei die gegeneinander schwenkbeweglichen Teile umschließender Arbeitsstellung einander benachbarten Längsrandbereiche
eines jeden Lagenabschnitts (2) miteinander durch Verschweißen, Verkleben, Verseifung oder dergleichen verbunden sind.

16.) Bandage nach einem der vorhergehenden Ansprüche, dadurch gekennzeichnet, daß längs beider Längsrandkonturen aller Lagenabschnitte (2) jeweils der Randstreifen (11) einer Hälfte einer teilbaren Verschlußleiste (10), wie beispielsweise eines teilbaren Reißverschlusses, Klettverschlusses oder Haken- und/oder Ösenverschlusses, festgelegt ist.

17.) Bandage nach Anspruch 16, dadurch gekennzeichnet, daß die Randstreifen (11) der beiden Hälften der teilbaren Verschlußleiste (10) jeweils unter Zwischenschaltung einer Longette (4) fixiermaterialfreien Materials, vorzugsweise Textilmaterials, festgelegt sind.

18.) Bandage nach Anspruch 16 oder 17, dadurch gekennzeichnet, daß die Randstreifen (11) der beiden Hälften der teilbaren Verschlußleiste (10) oder die Longetten (4) fixiermaterialfreien Materials jeweils durch den Aushärtungsvorgang des Fixiermaterials an diesem festgelegt sind.

19.) Bandage nach Anspruch 16 oder 17, dadurch gekennzeichnet, daß die Randstreifen (11) der beiden Hälften der teilbaren Verschlußleiste (10) oder die Longetten (4) fixiermaterialfreien Materials jeweils mechanisch am Trägerkörper des Fixiermaterials mindestens einer Lage (2), vorzugsweise aller Lagen, festgelegt sind.

20.) Bandage nach einem der Ansprüche 16 bis 19, dadurch gekennzeichnet, daß der durch den Randstreifen (11) der teilbaren Verschlußleiste (10) und/oder eine etwa zwischengeschaltete Longette (4) gebildete fixiermaterialfreie Streifenbereich eine einseitige Breite (b) von mindestens 2 cm vorzugsweise 3 cm, aufweist.

21.) Bandage nach einem der vorhergehenden Ansprüche mit Fixiermaterial aus Kunststoff, dadurch gekennzeichnet, daß das
Longettenmaterial aus durch chemische und/oder Vernetzungs-
Reaktion von Polyvinylalkohol mit Formaldehyd erhaltenem
Polyvinylalkoholformalschaum gebildet ist.

22.) Bandage nach einem der vorhergehenden Ansprüche mit Fixiermaterial aus Kunststoff, dadurch gekennzeichnet, daß der den
Bandagenrohling bildende Lagenabschnitt (2) durch auf ein
vorbestimmtes Wölbungs- und/oder Winkelkrümmungsmaß vorgeformtes Longettenmaterial gebildet ist.

23.) Bandage nach einem der vorhergehenden Ansprüche mit Fixiermaterial aus Kunststoff, dadurch gekennzeichnet, daß der Lagenabschnitt (2) aus vorzugsweise im Walzverfahren erstelltem
Kunststofflagenmaterial, vorzugsweise einer Kunststoffschaumlage, gebildet ist.

24.) Bandage nach einem der Ansprüche 1 bis 22 mit Fixiermaterial
aus Kunststoff, dadurch gekennzeichnet, daß der Lagenabschnitt
(2) ein Kunststoff- oder Kunststoffschaum-Gießling vorbestimmter Wölbungs- und/oder Winkelkrümmungskontur ist.

Patentanwalt

*Fig.1*

0006607

_Fig.2_

**Europäisches Patentamt**

## EUROPÄISCHER RECHERCHENBERICHT

EP 79 102 112.4

| | **EINSCHLÄGIGE DOKUMENTE** | | **KLASSIFIKATION DER ANMELDUNG (Int.Cl.3)** |
|---|---|---|---|
| **Kategorie** | **Kennzeichnung des Dokuments mit Angabe, soweit erforderlich, der maßgeblichen Teile** | **betrifft Anspruch** | |
| X | <u>US - A - 4 019 505</u> (L.H. WARTMAN) <br> * Spalten 1 bis 3; Spalte 5, Zeilen 52 bis 68; Spalte 6; Fig. 2 * <br> -- | 1,3,5, 6,12, 15,22, 23 | A 61 F 13/04 <br> A 61 L 15/07 |
| X | <u>US - A - 2 512 081</u> (L.R. YOUNG) <br> * Spalten 1 bis 3; Fig. 1,2 * <br> -- | 1,4, 8-14 | |
| X | <u>DE - A - 2 509 933</u> L.E. LAUBER et al.) <br> * Ansprüche 1, 10, 13; Seite 3, Zeilen 2 bis 20; Seite 7, Absatz 2; Fig. 1 bis 4 * <br> -- | 1,2,4 | **RECHERCHIERTE SACHGEBIETE (Int. Cl.3)** <br><br> A 61 F 5/04 |
| X | <u>DD - A - 126 689</u> (YARDNEY COMPANY) <br> * Seite 36, letzter Absatz bis Seite 37, Absatz 1; Seite 48, Absatz 2; Fig. 3,4 * <br> -- | 1,3, 5-8, 22,23 | A 61 F 13/00 <br> A 61 L 15/07 |
| X | <u>AT - B - 311 545</u> (BAKELITE XYLONITE LTD.) <br> * Seite 7; Fig.1,3,5 * <br> -- | 1,3,5, 6,15 | |
| P,X | <u>DE - U - 7 834 078</u> (EUROCOM) <br> * ganzes Dokument * <br> -- | 1-24 | **KATEGORIE DER GENANNTEN DOKUMENTE** |
| P | <u>DE - U - 7 819 199</u> (EUROCOM) <br> * ganzes Dokument * <br> -- <br><br> ./... | 1-24 | X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument <br> &: Mitglied der gleichen Patentfamilie, übereinstimmendes Dokument |

| X | Der vorliegende Recherchenbericht wurde für alle Patentansprüche erstellt. |
|---|---|

| **Recherchenort** <br> Berlin | **Abschlußdatum der Recherche** <br> 12-09-1979 | **Prüfer** <br> DROPMANN |
|---|---|---|

EPA form 1503.1 06.78

Europäisches
Patentamt

**EUROPÄISCHER RECHERCHENBERICHT**

0006607

Nummer der Anmeldung

EP 79 102 112.4
−Seite 2−

| | **EINSCHLÄGIGE DOKUMENTE** | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| P | DE − U − 7 819 206 (EUROCOM) <br><br> * ganzes Dokument * <br><br> −− | 1−24 | |
| | US − A − 3 538 914 (H.L. MYERS) <br><br> * Fig. 1 * <br><br> −− | 16,19 | |
| | DE − A − 2 618 613 (TEMCA CHEMISCHE UNION) <br><br> * Seite 3 * <br><br> −−−− | 21 | |
| | | | RECHERCHIERTE SACHGEBIETE (Int. Cl.3) |